# EUROPEAN PATENT SPECIFICATION

(11) **EP 2 114 527 B1**
(45) Date of publication and mention of the grant of the patent: **22.08.2018**
(21) Application number: 07835059.2
(22) Date of filing: 27.09.2007
(51) Int. Cl.: A61N 1/372, A61B 5/0205, A61B 5/145, A61B 5/11

(54) **DEVICE AND METHOD OF A MEDICAL IMPLANT FOR MONITORING PROGRESSION OF HEART FAILURE IN A HUMAN HEART**
VORRICHTUNG UND VERFAHREN FÜR EIN MEDIZINISCHES IMPLANTAT ZUR ÜBERWACHUNG DES FORTSCHREITENS VON HERZINSUFFIZIENZ IN EINEM MENSCHLICHEN HERZ
DISPOSITIF ET PROCÉDÉ D'IMPLANT MÉDICAL POUR SURVEILLER LA PROGRESSION D'UNE DÉFAILLANCE CARDIAQUE DANS UN COEUR HUMAIN

(30) Priority: 28.02.2007 WO PCT/SE2007/000192
(43) Date of publication of application: 11.11.2009
(73) Proprietor: St. Jude Medical AB, 175 84 Järfälla (SE)
(72) Inventor: BJÖRLING, Anders, 16937 Solna (SE); ÖHLANDER, Malin, 11848 Stockholm (SE); ERIKSSON, Tom, 81132 Sandviken (SE); ECKERDAL, Johan, 741 92 Knivsta (SE); LÖNN, Urban, 756 45 Uppsala (SE); NILSSON, Kenth, 18460 Åkersberga (SE); TUVSTEDT, Cecilia, 113 24 Stockholm (SE); SVAHN, Johan, 168 67 Bromma (SE); JOHANSSON, Anna-Karin, 18696 Vallentuna (SE); NOREN, Kjell, 17158 Solna (SE); BROOME, Michael, 17838 Ekerö (SE)
(74) Representative: Sjölander, Henrik
(86) International application number: PCT/SE2007/000855
(87) International publication number: WO 2008/105698

(56) References cited:
- EP-A1- 0 467 213
- WO-A1-2007/011565
- WO-A1-2007/011565
- US-A- 5 891 176
- US-A- 6 162 180
- US-A1- 2003 149 367
- US-A1- 2003 149 367
- US-A1- 2003 199 956
- US-A1- 2003 199 956
- US-A1- 2003 220 582
- US-A1- 2004 147 982
- US-A1- 2004 147 982
- US-B1- 6 190 324
- US-B1- 6 198 952
- A. Ohlsson ET AL: "Continuous ambulatory haemodynamic monitoring with an implantable system The feasibility of a new technique", European Heart Journal, 1 January 1998 (1998-01-01), pages 174-184, XP55000880, Retrieved from the Internet: URL:http://eurheartj.oxfordjournals.org/co ntent/19/1/174.full.pdf [retrieved on 2011-06-17]
- KJLLSTRöM B. ET AL.: 'Six years follow-up of an implanted Sv0(2) sensor in the right ventricle' EUROPEAN JOURNAL OF HEART FAILURE vol. 6, no. 5, August 2004, pages 627 - 634, XP003022754
- LAU C.-P. ET AL.: 'Utility of an implantable right ventricular oxygen saturation-sensing pacemaker for ambulatory cardiopulmonary monitoring' CHEST vol. 107, no. 4, 1995, pages 1089 - 1094, XP003022755
- MCELROY P.A. ET AL.: 'Physiologic correlates of the heart rate response to upright isotonic exercise: Relevance to rate-responsive pacemakers' JOURNAL OF THE AMERICAN COLLEGE OF CARDIOLOGY vol. 11, no. 1, 1988, pages 94 - 99, XP003022756

## Description

### Technical field

The present invention generally relates to the field of implantable heart stimulation devices, such as pacemakers, implantable cardioverter-defibrillators (ICD), implantable cardiac monitors and similar cardiac stimulation devices. More specifically, the present invention relates to a device in a medical implant for monitoring the progression of heart failure in a human heart, a cardiac implant including such a device, a method for monitoring the progression of heart failure in a human heart, and a computer program product.

### Background art

Between 20 and 25 million people worldwide are afflicted with congestive heart failure (CHF), and 2 million new patients are diagnosed each year. In contrast to other cardiovascular disorders that have declined over the past few decades, the incidence of congestive heart failure is on the rise. Congestive heart failure, also called congestive cardiac failure (CCF) or just heart failure (HF), is a condition that can result from any structural or functional cardiac disorder that impairs the ability of the heart to fill with or pump a sufficient amount of blood throughout the body.

The New York Heart Association (NYHA) Functional Classification provides a simple way of classifying the extent of heart failure. It places patients in one of four categories based on how much they are limited during physical activity:
I No symptoms and no limitation in ordinary physical activity.
II Mild symptoms and slight limitation during ordinary activity. Comfortable at rest.
III Marked limitation in activity due to symptoms, even during less-than-ordinary activity. Comfortable only at rest.
IV Severe limitations. Experiences symptoms even while at rest.

There is no cure for CHF, short of a heart transplant. Although advances in pharmacology have led to better treatment, about 50 % of the patients with the most advanced stage of heart failure die within a year. A majority of patients are treated with drug therapy, such as chronic oral therapies including diuretics, ACE inhibitors, Beta-blockers and inotropic agents. However, for patients with advanced stages of CHF, device-based therapy, i.e. cardiac stimulators, or transplants are the only alternatives.

In clinical health care of intensive care patients, the degree of heart failure of myocardial infarction patients, and patients experiencing other cardiac disorders, is suitably monitored. One method of clinically monitoring heart failure uses SvO₂ values measured using catheters in the pulmonary artery. It is considered as a reliable and important tool in this situation, see for example "Central venous oxygen saturation monitoring in the critically ill patient", Rivers et al., Curr Opin Crit Care, June 2001; 7(3): 204-211. Review. PMID: 11436529.

SvO₂ and SaO₂ are physiological parameters that has long been used by physicians to diagnose and monitor patients. SaO₂ (arterial oxygen saturation) is the percentage of hemoglobin binding sites that is occupied by oxygen in the arterial bloodstream, while SvO₂ (mixed venous oxygen saturation) is the corresponding percentage in the venous blood after mixing. In other words, the difference between SaO₂ and SvO₂ corresponds to the oxygen consumption by the bodily tissue. Therefore, SvO₂ varies directly with cardiac output and SaO₂ and inversely with oxygen consumption. A normal SaO₂ value in a healthy subject is about 97 % and a normal SvO₂ value is about 70% at rest, and varies with the activity level of the subject. In other words, under normal conditions, tissues extract about 25% of the oxygen delivered. An increase in oxygen consumption or a decrease in arterial oxygen content (SaO₂ x Hb) is compensated by increasing cardiac output or the oxygen extraction by the tissue. Even though a normal SvO₂ does not ensure a normal metabolic state, but suggests that oxygen kinetics are either either normal or compensated. SvO₂ is thus a global parameter indicating how well oxygenated the body is.

When a healthy subject starts to exercise, there is an almost immediate drop in the SvO₂ value, see for example "Abrupt changes in mixed venous blood gas composition after the onset of exercise", Casaburi et al., J Appl. Physiol., September 1989, 67(3):1106-1112, as well as "Continuous monitoring of mixed venous oxygen saturation during exercise using fiberoptic pulmonary catheter", Nakanishi et al., Kokyo To Junkan; August 1990; 38(8):799-804. Thus, the body reacts to the increased oxygen demand both by increasing cardiac output, i.e. increasing heart rate and stroke volume, and by extracting more oxygen from each unit of blood volume. Since the SaO₂ level remains fairly constant, the increased oxygen extraction results in a decreased SvO₂ value. Consequently, the patient must be at rest in order for variations of the patent activity level not to affect the evaluation of the SvO₂ values.

However, measurements of SvO₂ often uses blood samples from the pulmonary artery and monitoring of heart failure using SvO₂ is therefore generally performed in a clinical setting, typically in an intensive care unit where the level of physical activity or exercise is not a factor. In other words, the SvO₂ measurements are then performed with the patient at rest.

Accordingly, a need for monitoring the appearance, progression and possible regression of heart failure, as well as detecting advanced stages of heart failure, away from the intensive care unit remains to exist. "Continuous ambulatory haemodynamic monitoring with an implantable system", Ohlsson et al, European Heart Journal, 1998, 19:174-184 discloses an implantable system for long-term ambulatory monitoring of haemodynamic parameters, including activity, heart rate, mixed venous oxygen saturation and estimated pulmonary artery diastolic pressure, in patients with severe cardiopulmonary disease. The parameters could be used to determine cardiac output of the patients.

### Summary of the invention

An object of the present invention is to provide a solution for continuous monitoring of the progression of heart failure.

This and other objects are achieved by a device as claimed in the independent claims. Further embodiments are defined in the dependent claims.

According to a first aspect, there is provided a device in a medical implant for monitoring progression of heart failure in a human heart. The device is connectable to an oxygen sensor and an activity sensor, said oxygen sensor being sensitive to a level of venous oxygen content, and arranged to output an oxygen signal, and said activity sensor being sensitive to a level of physical activity of a patient and arranged to output an activity signal. The device comprises processing circuitry arranged for receiving an oxygen signal from said oxygen sensor, receiving an activity signal from said activity sensor, determining the level of physical activity from the received activity signal, comparing at least one parameter of the received oxygen signal to at least one corresponding stored oxygen signal parameter for the determined level of physical activity, and determining the degree of heart failure on the basis of said comparison.

According to a second aspect, there is provided an implantable cardiac stimulator for delivering stimulation pulses to a human heart. The cardiac stimulator comprises a pulse generator for generating said stimulation pulses, and control circuitry for controlling the delivery of said stimulation pulses to the heart. The stimulator is connectable to a cardiac lead arrangement for conduction of stimulation pulses to the heart and sensed cardiac electrical activity from the heart, said lead arrangement further being arranged for sensing atrial electrical activity and atrial mechanical activity. Furthermore, the stimulator comprises a device according to the first aspect.

Thus, the present invention is based on the insight of using an activity sensor and an oxygen sensor in a medical implant for monitoring the progression of heart failure or congestive heart failure. By obtaining an oxygen signal indicative of a level of venous oxygen content, and also determining a level of physical activity of a patient carrying the implant, parameters of the obtained oxygen signal can be compared to stored parameters at the same or similar level of physical activity for the patient. Thereby, a reliable determination of the degree of heart failure can be continuously determined for a patient away from a clinical setting.

Monitoring the long term progression and regression of heart failure is of essence for the physician to assess the patient status, titrating drugs, evaluating therapy, and to make therapeutic decisions for the patients. Also, having parameters such as SvO₂ monitored continuously, and not just measurements taken in the hospital, would provide a better and more complete picture of the progression of heart failure.

The determined level of heart failure degree may be stored for later use by the physician at clinical follow-ups. Then, a number of measurements and results of degree of heart failure determinations may be stored such that a trend over time may be displayed to or deduced by the physician. Also, the measurements of compared oxygen signal parameters, as well as activity signal parameters, may also be stored, processed or unprocessed, for subsequent evaluations at follow-ups or the like.

In preferred embodiments of the invention, the stored oxygen signal parameters that are to be used in comparison with present oxygen signal parameters, and for determining degree of heart failure, have been obtained through previous measurements for the same patient. Thus, even though theoretical parameter values may be used for said comparisons, use is preferably made of patient specific parameters. Thereby, higher sensitivity and determination specificity may be obtained.

In further embodiments, the stored oxygen signal parameters are updated with later measurements such that the stored set of oxygen signal parameters may reflect the current status, as well as the historic status for the patient. Thereby, the progression of heart failure may be monitored over time. Preferably, this is performed for one or more corresponding levels of physical activities, which level of physical activity could include the patient being at rest.

In exemplifying embodiments, a measurement of physical activity is performed, followed by a determination of whether the patient is at rest or is exercising. The procedure of determining may then be continued only if it is determined that the patient is at rest, or alternatively only if it is determined that the patient is not at rest. However, it is preferred that measurements can be made regardless of whether the patient is at rest or not.

An advantage of only using measurements at rest for monitoring the progression of heart failure is that it the measurement conditions vary very little. Thus, the results of measurements performed at separate instances may be easily compared with a high degree of specificity. However, a drawback of only using measurements at rest is that the variations in the oxygen signals are small and/or only appear at more severe degrees of heart failure. Thus, it would be difficult or very difficult to determine progression of heart failure in patients in NYHA Class I and II, which would make measurements at rest unsuitable for monitoring progression of heart failure for such patients. However, for patients with a degree of heart failure corresponding to NYHA Class III or IV, measurements at rest would most likely suffice. Furthermore, measurements at rest would likely be more advantageous than measurements at exercise for such patients, due to their difficulty in performing physical activities.

As understood from the above, measurements during periods of physical activity or exercise is advantageous due to the greater variations in oxygen signal parameters for mixed venous blood. Thus, as long as the level and/or type of physical activity can be controlled, measured and determined, the degree of heart failure can be determined with a higher degree of sensitivity and specificity at exercise than at rest. Thus, measurements and evaluations of oxygen signals related to venous blood and performed during exercise is particularly advantageous for patients with less severe degrees of heart failure, such as for patients in NYHA class I and II. However, since the oxygen signal parameters in venous blood varies significantly with physical activity, the specificity may be reduced if the level of physical activity can not be determined and the oxygen measurements is not correlated with the level of physical activity at the time of measurement.

In exemplifying embodiments of the invention, the level of physical activity is determined, and parameters of the oxygen signal obtained during the period of exercise is used for the comparison to stored parameters. Since there are a number of different oxygen parameter features that can be evaluated during exercise, the oxygen signal is preferably monitored during a time period including one or more of:
from onset of exercise until a stable oxygen signal during exercise, which time is affected by progressive heart failure,
the time from the end of exercise during which a stable oxygen level has been reached until the oxygen signal has returned to a normal value,
from the end of exercise to a predefined time thereafter, for monitoring level of overshoot as will be explained further below, which is affected by progressive heart failure, and
from the end of exercise to a return of oxygen signal to a stable value corresponding to that of rest prior to the exercise, which time is affected by progressive heart failure.

For the latter alternatives, reference is made to "Overshoot in mixed venous oxygen saturation during recovery from supine bicycle exercise in patients with recent myocardial infarction", Sumitomo et al., Chest, February 1993, 103(2): 414-520. In this document, it is disclosed that there is an overshoot of the SvO₂ value after the completion of exercise. In other words, the SvO₂ value was higher 2 minutes and 5 minutes after exercise then the SvO₂ value at rest before the exercise. This is also supported by the study presented in "Prolonged recovery of cardiac output after maximal exercise in patients with chronic heart failure", J Am Coll Cardiol. April 2000; 35(5):1228-1236. In Sumitomo et al. it was also found that the amplitude of the SvO₂ overshoot correlated with the degree of heart failure in accordance with the NYHA Functional Classification. Thus, it would be of interest to monitor the oxygen signal after end of exercise until the oxygen signal has become stable at the level of patient rest.

In exemplifying embodiments, the determined level of physical activity is assessed for determining that it is suitable for performing said measurements, comparison and heart failure determination.

The measurements of physical activity may in some embodiments be performed continuously for triggering heart failure determination when the determined level of physical activity is suitable. Then, a waiting period may be initiated immediately following a heart failure degree determination. In other embodiments, the heart failure determination may be performed at specific intervals, such as once a day, once a week, or once a month. It may also be patient initiated, or performed at follow-ups in a clinical setting at time selected by a physician.

Furthermore, in different embodiments, the measurements may be performed on a patient manual basis, on a semi-automatic basis, or on a fully automatic basis. In the patient manual alternative, the patient would manually trigger a determination of heart failure degree. Then, the patient could follow a specific activity scheme, such as walking on a treadmill at a given pace, cycling at a determined pace with a controlled resistance, walking a specified route, doing predefined arm and/or leg movements, etc. Alternatively, the implant could be taught to recognize and discriminate between different types of activities, while still being triggered to initiate measurements by the patient.

In embodiments of semi-automatic determination of degree of heart failure, a calibration process could be performed at a clinical setting that would teach the implant to recognize certain physical activities. Then, the implant could monitor the physical activities performed by the patient and initiate a heart failure degree determination when the monitored activity concurs with one of the calibrated activities.

In fully automatic embodiments, a determination of degree of heart failure is automatically initiated, and the measured level of activity and measured oxygen signal parameters are used regardless of the type of activity. However, the determined activity level is preferably stored with the measured oxygen signal, such that subsequent measurements may be compared to stored measurements for a specific activity level. Preferably, measured levels of activity are classified into groups, such that comparisons of oxygen signal parameters are only performed within the same group of physical activity.

The determination of level of activity can be based on different features of an activity signal, such as amplitude, frequency, morphology, etc. Furthermore, the determination and possible classification of activity levels may be adapted to previously determined levels of activity.

In embodiments of the invention, the oxygen signal is a signal representative of the SvO₂ value, i.e. the oxygen saturation level in mixed venous blood. Consequently, the oxygen sensor is in embodiments of the invention an SvO₂ sensor sensitive to the levels of oxygen in venous blood. However, the SvO₂ and SaO₂ values are correlated with the partial pressure of oxygen, pO₂, in blood. In particular, for the levels of oxygen saturation that are present in mixed venous blood, for example SvO₂ levels between 25% and 75%, the correlation with pO₂ assumes an essentially linear function. In other words, SvO₂ values may easily be calculated from measured pO₂ values, and vice versa. Thus, in embodiments of the invention, the oxygen signal is a pO₂ signal, and the oxygen sensor a pO₂ sensor, for example, a sensor as described in US 5,431,172.

As a further example, the oxygen signal may be an pO₂ signal from a pO₂ sensor. According to another embodiment, the oxygen signal may be an SvO₂ signal calculated from the measurement signal obtained from a sensor utilizing photoluminescent molecules emitting light as a response to the concentration of oxygen in blood.

In yet another embodiment, the oxygen signal may be a pO₂ signal from a sensor that electrochemically measures the concentration of oxygen in blood as described in US 6,236,873.

In embodiments of the invention, the activity sensor is in the form of or comprises one or more accelerometers. Examples of accelerometers that may be used for obtaining an activity signal in accordance with the present invention are provided in US Patents Nos. 5,183,056; 5,425,750; 5,496,352; and 6,466,821, which are all incorporated herein by reference in their entirety. However, as understood by the skilled person, any implantable accelerometer suitable for measuring and detecting activity levels of a patient may be used without departing from the scope of the present invention.

In addition to using accelerometers, or as alternatives thereto, as activity sensors, use can be made of physiological sensors indicative of the level of activity for a patient. Examples of such sensors include sensing of QT interval and controlling the pacing rate in response to variations of the QT interval, and sensing respiratory minute volume and controlling the pacing rate dependent on respiratory minute volume.

Furthermore intrinsic rate for patients who are not chronotropically incompetent can be used as a measure of activity.

Furthermore, in embodiments of the invention, the oxygen sensor is in the form of an optical sensor sensitive to the SvO₂ levels of a patient. An example of such a sensor will be described in more detail below. Further examples of oxygen sensors that may be used for obtaining an oxygen signal in accordance with the present invention are provided in US Patents Nos. 4,815,469; 5,614,246; 5,728,281; 6,236,873; and 6,321,101, which are all incorporated herein by reference in their entirety. However, as understood by the skilled person, any oxygen sensor suitable for implantation such that it may be subjected to mixed venous blood may be used without departing from the scope of the present invention.

According to exemplifying embodiments, in addition to determining degree of heart failure of a patient, the determined heart failure degree may also give rise to alert signals alerting the patient and/or the physician that an action must be performed. Such actions could include conducting a clinical follow-up, transmitting information to a physician for evaluation, instructing the patient to contact his/her physician, or alerting the patient to seek immediate medical assistance. Then, use can be made of a vibrating alarm device within the medical implant, a telemetry communication unit within the medical implant, an extracorporeal communication unit such as a programmer or a home monitoring unit, a wireless communication system for communication with a clinic, etc., all of which being well known in the art and not discussed herein in further detail.

Further objects and advantages of the present invention will be discussed below by means of exemplifying embodiments.

### Brief description of the drawings

Exemplifying embodiments of the invention will be described below with reference to the accompanying drawings, in which:
Fig. 1 schematically shows an embodiment of a pacemaker system in which a device and method may be implemented;
Fig. 2 schematically shows a medical implant;
Fig. 3 schematically shows a medical system in accordance with an embodiment including the implantable medical device shown in Fig. 2;
Fig. 4 shows an optical sensor module which may be implemented in a medical lead connectable to the device and medical implant;
Fig. 5 illustrates the principles of oxygen saturation measurements using the sensor module of Fig. 4;
Fig. 6 illustrates examples of SvO₂ features that can be measured at the occurrence of patient physical activity; and
Figs. 7-9 are flow diagrams illustrating on high-level basis various methods according to exemplifying embodiments.

### Detailed description of exemplifying embodiments

The following is a description of exemplifying embodiments in accordance with the present invention. This description is intended for describing the general principles and specific embodiments of the invention and is not to be taken in a limiting sense. Thus, even though a dual chamber heart stimulator will be described, the invention is also applicable to biventricular stimulators with both right and left ventricular stimulation. Furthermore, the invention is not restricted to pacemakers, but to any sort of cardiac stimulators, such as implantable cardioverter defibrillators (ICD). The invention can also be utilized in implantable monitoring units that only collect diagnostic data with no therapeutic functionality. Please note that like reference numerals indicate structures or elements having same or similar functions or constructional features.

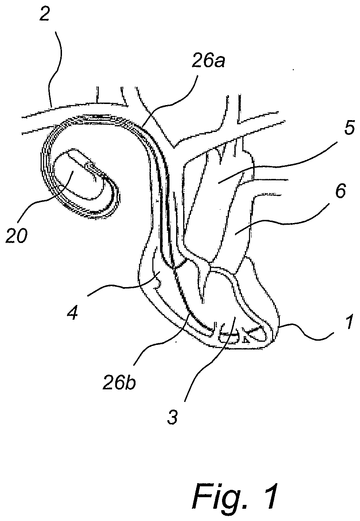

With reference to Fig. 1, there is shown a schematic diagram of a medical implant in which the present invention can be implemented. This embodiment of the present invention is shown in the context of a pacemaker 20 implanted in a patient. The pacemaker 20 comprises a hermetically sealed and biologically inert housing, which normally is conductive and may serve as an electrode. One or more pacemaker leads are electrically coupled to the pacemaker 20 in a conventional manner. In this embodiment, the pacemaker leads 26a, 26b extend into the heart 1 via a vein 2 of the patient and include a ventricular lead 26a implanted in the right ventricle 3 and an atrial lead 26b implanted in the right atrium 4, as shown in Fig. 1. One or more conductive electrodes for receiving electrical cardiac signals and/or for delivering electrical pacing to the heart 1 are arranged near the distal ends of the leads 26a, 26b. As the skilled man in the art realizes, the leads 26a, 26b may be implanted with its distal end located in either the ventricle 3 or atrium 4 of the heart 1.

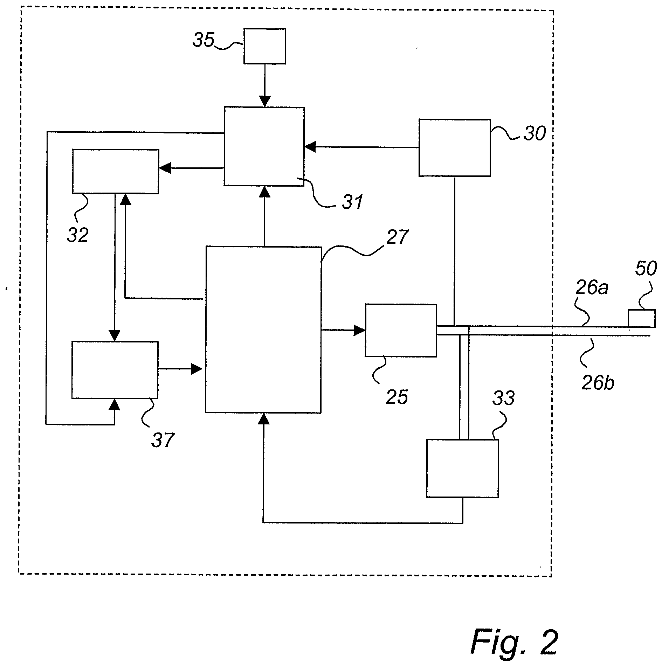

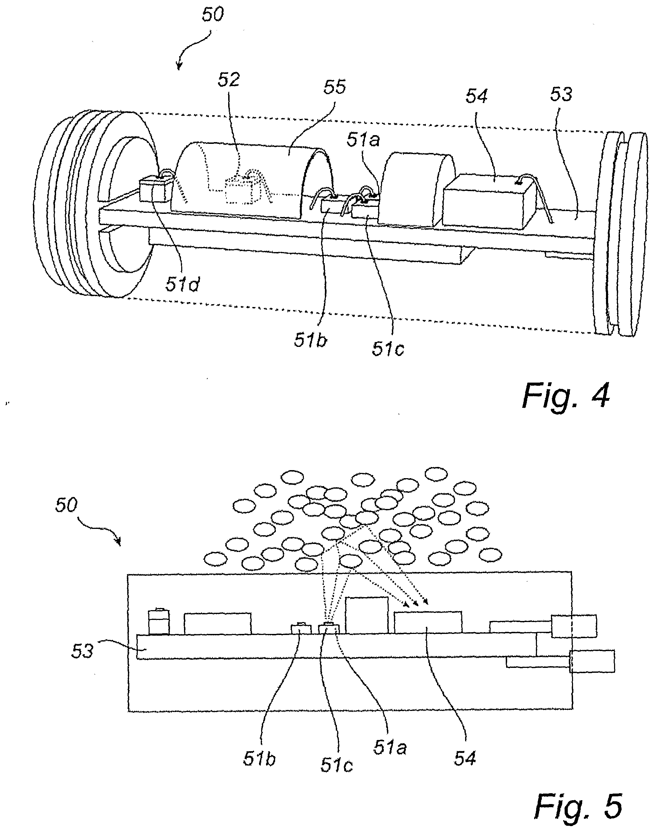

With reference now to Fig. 2, the configuration including the primary components of an embodiment of the present invention will be described. The illustrated embodiment comprises the medical implant or pacemaker 20, and the leads 26a and 26b for delivering electrical pulses and signals between the medical implant 20 and the heart. The leads 26a, 26b may be unipolar or bipolar, and may include any of the passive or active fixation means known in the art for fixation of the lead to the cardiac tissue. As an example, the lead distal tip (not shown) may include a tined tip or a fixation helix. The leads 26a, 26b comprise one or more electrodes (as mentioned with reference to fig. 1), such a tip electrode or a ring electrode, arranged to, inter alia, transmit pacing pulses for causing depolarization of cardiac tissue adjacent to the electrode(-s) generated by a pace pulse generator 25 under influence of a control circuit 27 comprising a microprocessor. The control circuit 27 controls, inter alia, pace pulse parameters, such as output voltage and pulse duration. An optical sensor module 50, which will be discussed in more detail with reference to Fig. 4, is further arranged in the atrial lead 26b and arranged to measure and determine a mixed venous oxygen saturation, i.e. SvO₂, level of the blood.

Furthermore, the optical sensor module 50 is connected to a blood constituent determining device 30 adapted to obtain measured SvO₂ values from the optical sensor module 50, to determine a present SvO₂ level by means of the at least one SvO₂ value. The SvO₂ values may be obtained at regular basis, i.e. at regular intervals, or continuously. Thereby, it is possible to obtain a sequence over time of SvO₂ values.

A patient status determining device 31 is connected to the blood constituent determining device 30 and is adapted to determine a patient status based on an evaluation of the present hematocrit level and the present SvO₂ level. The patient status may be used to derive a change of a condition of the patient. The implant 20 further comprises an activity level sensor 35 connected to the patient status determining device 31. In this embodiment, the activity level sensor 35 comprises at least one accelerometer arranged within the casing of the implant. However, activity level sensors arranged externally of the implant casing are also contemplated. Furthermore, other activity level sensors than accelerometers are also contemplated, such as QT interval detection sensors or minute ventilation sensors. Thus, the patient status determination device 31 is capable of obtaining information on venous oxygen levels derived from the optical sensor 50, and on activity levels from the activity sensor 35.

It should be noted that the patient determining device 31 may be connected to other physiological sensors for determining a status of the patient, as well as to sensors for sensing intrinsic and evoked atrial and/or ventricular heart beats. Thus, the patient status determination device 31 could be provided with the ability of obtaining information on other physiological parameters such as body temperature, heart rate, patient posture, etc. However, for the purposes of the presently described embodiment, the patient status determination device 31 is arranged for receiving activity and oxygen information from the activity level sensor 35 and the optical sensor 50.

Detected signals from the patients heart are processed in an input circuit 33 and are forwarded to the microprocessor of the control circuit 27 for use in logic timing determination in known manner. The implantable medical device 20 is powered by a battery (not shown), which supplies electrical power to all electrical active components of the medical device 20. Data contained in, for example, the memory circuit of the control circuit 27, the patient status determining device 31, or the therapy determining device 32 can be transferred to an extracorporeal device, such as a programmer (not shown), via a programmer interface (not shown) and the telemetry communication unit 37. The date could then be used for analyzing system conditions, patient information, etc. The telemetry communication circuit 37 is adapted for two-way communication with at least one extracorporeal device including a communication unit.

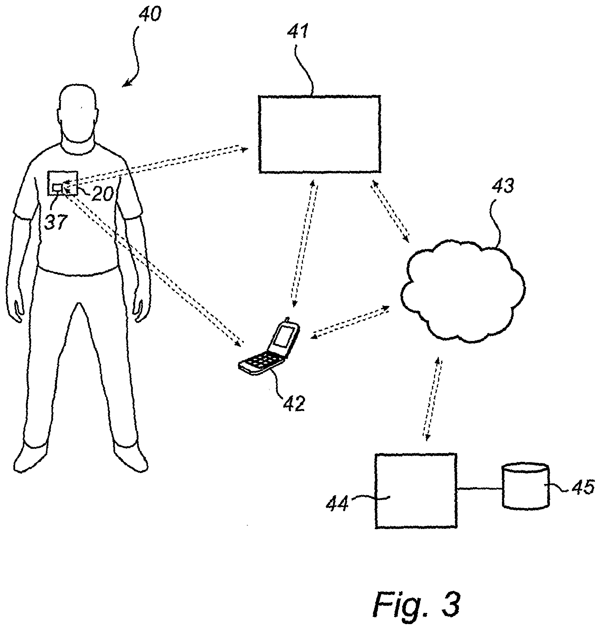

With reference now to Fig. 3, an exemplifying system environment will be discussed. An implantable medical device 20 as described above with reference to Fig. 2 is implanted in a patient 40. As discussed above, the medical implant may transfer data, such as a patient status or a change in the monitored degree of heart failure, or any other monitored physiological parameter, to extracorporeal devices 41, 42, 44 via the RF communication unit 37. The extracorporeal devices 41, 42, 44, may communicate with each other via at least one external communication network such as wireless LAN ("Local Area Network"), GSM ("Global System for Mobile communications"), UMTS ("Universal Mobile Telecommunications System"). For a given communication method, a multitude of standard and/or proprietary communication protocols may be used. For example, and without limitation, wireless (e.g. radio frequency pulse coding, spread spectrum frequency hopping, time-hopping, etc.) and other communication protocols (e.g. SMTP, FTP, TCP/IP) may be used. Other proprietary methods and protocols may also be used.

The communication unit 37 is adapted for two-way communication with an extracorporeal home monitoring unit 41, which may be located in the patients home, including a display means such as a display screen and input means such as a mouse and a keyboard and/or a user equipment 42, such as a mobile phone, a personal digital assistant, or a pager. Furthermore, the user equipment 42 may be adapted to be carried by the patient similar to a wrist watch or to be attached at a belt. The communication unit 37 may also communicate with a monitoring device 44, e.g. a PC, located at, for example, a care institution via the home monitoring unit 41 and/or via the user equipment 42 via a communication network as described above or via Internet. The monitoring device 44 may be connected to a database 45 for storage of patient data.

In embodiments of the present invention, the patient status determining device 31 may transfer patient status data and/or trend data of the different measured parameters, including SvO₂ and corresponding activity levels, to the extracorporeal devices 41, 42, 44 via the telemetry communication unit 37. As the skilled person realizes, there are other physiological/hemodynamical parameters that may be monitored, such as cardiovascular pressure, cardiac output, PR interval (or AR interval), hematocrit, body temperature, heart rate, patient posture and/or minute ventilation. This information may also be transferred to the monitoring device 44 at the care institution via the communication network 43, either directly or via the home monitoring unit 41 or the user equipment 42, thereby allowing a physician to view a progression/regression of heart failure, as well as other diseases, and/or a trend of a certain parameter or certain parameters. The trend may either be displayed to the physician at a follow-up of the patient or upon an inquiry sent to the implantable medical device 20 from the monitoring device 44 via the communication network 43 and the home monitoring unit 41. The information can be used to guide long term therapy, such as if the patient should be equipped with a different device or if the type of medication should be changed. The information may also be used by the physician to determine a dosage of a drug.

Furthermore, predetermined upper of lower limits may be set for SvO₂ levels, per se and/or correlated with a particular exercise or physical activity level, within which SvO₂ levels are allowed to fluctuate between. Each criterion may give rise to an alert signal. The patient status determining device 31 may send such an alert signal to the user equipment 42 and/or the home monitoring unit 41 informing the patient that he or she should see his/her physician. In another embodiment, the medical device 20 may include an alarm means adapted to cause the device to vibrate or to deliver a beeping sound in order to alert the patient of the situation, the alarm means may be integrated into the control circuit 27 or the patient status determining device 31. Alternatively, or as a complement, this information together with the progression of the trend may be sent with an alert signal to the physician to be viewed on the monitoring device 44 so that he or she can decide whether the patient should be called in for a visit.

Referring now to Fig. 4, the optical sensor module 50 will be described. The sensor module is based on the different light reflecting properties of oxygenated and reduced hemoglobin. The measurements are influenced by, inter alia, hematocrit level. The use of two or more wavelength may compensate for this effect. The optical sensor module 50 is integrated in a medical lead, for example, the atrial lead and is hermetically sealed inside a tube, for example, of sapphire. According to an embodiment, four LEDs 51a, 51b, 51c, 51d at wavelengths 670, 700, 805, and 805 nm, respectively, and a built in calibration photodiode 52 are arranged on a substrate 53 in the module 50. Furthermore, a photodiode 54 is adapted to receive the light emitted from the LEDs 51a, 51b, 51c, 51d and reflected at a reflective surface 55. According to this embodiment, the first, second, third LED 51a, 51b, and 51c are adapted to emit light at wavelengths 670, 700, and 805 nm to measure oxygen saturation (SvO₂), which is schematically illustrated in Fig. 5. The theoretical background of the optical sensor and of the different light reflecting properties of oxygenated and reduced hemoglobin as well as the influence of, inter alia, hematocrit level, blood flow and erythrocyte shape on the measurements are described in detail in US patent No. 4,114,604, thereby well known to the skilled person and therefore not repeated here in further detail.

The device for monitoring progression of heart failure in a human heart according to the present invention, may comprise other types of sensors instead of, or as a complement to, the optical sensor described above.

In one embodiment, a pO₂ sensor and the control circuit 27 may be arranged for calculating an pO₂ signal out of the received oxygen signal. In US 5,431,172 a sensor for measuring a partial pressure of gases, for example, oxygen dissolved in blood of a patient is described, which sensor may be arranged in the device for monitoring progression of heart failure in a human heart according to the present invention.

Furthermore, according to another embodiment, a sensor for measuring the concentration of oxygen in blood by means of photoluminescence is arranged in the device for monitoring progression of heart failure in a human heart according to the present invention. Such a sensor is described in PCT/SE2007/000410 ("IMPLANTABLE DEVICES AND METHOD FOR DETERMINING A CONCENTRATION OF A SUBSTANCE AND/OR MOLECULE IN BLOOD OR TISSUE OF A PATIENT). The sensor comprises a carrier in which photoluminescent molecules are embedded. The carrier is partially in contact with blood of a patient comprising oxygen for which the concentration shall be determined. The sensor further comprises a light source and a photo-detector which are optically connected to the carrier such that the light emitted from the light source can excite the photoluminescent molecules and such that the light emitted from the photoluminescent molecules in response to this excitation can be detected by the photo-detector. The carrier is selectively permeable to the oxygen molecules for which the concentration has to be determined such that oxygen molecules can diffuse from the region of the patient into the carrier. The photoluminescent molecules are selected to react with oxygen in the blood in such a manner that the characteristics of the light emitted from the photoluminescent molecules is altered because of the reaction. Depending on the concentration of oxygen molecules in the blood of the patient, the characteristics of the light emitted from the photoluminescent molecules will then be altered to different degrees. Thus, a determination of the concentration of oxygen in the blood of the patient is enabled.

In an embodiment, the light source and the photo-detector are directly arranged at a side of the carrier or in the carrier. In another embodiment, the light source and the photo-detector are optically connected to the photoluminescent molecules comprised in the carrier by means of optical fibers. The optical fibers may be arranged between the light source and the photoluminescent molecules and/or between the photo-detector and the photoluminescent molecules, which means that the light source and the photo-detector can be arranged at a certain distance from the analyzed region. The optical fibers are used to guide light from the light source and to guide light to the photo-detector, respectively.

In an embodiment, the light source and the photo-detector are fabricated on a same substrate using e.g. standard semiconductor technology. The carrier is then made of a material, such as a silicon adhesive, like e.g. the commercially available Rehau RAU-SIK SI-1511, containing the photoluminescent molecules and spread over the substrate. The substrate can then be used as a support for the light source, the photo-detector and the carrier. Such a substrate would also enable easy connection between the light source, the photo-detector and other components used to analyze the signal output of the photo-detector. The thickness of the adhesive film containing the photoluminescent molecules would typically be comprised between 0.03 and 3 mm.

In yet a further embodiment, the light source and the photo-detector can be fabricated on two separate substrates and joined together by means of the carrier, e.g. the silicon adhesive mentioned above, containing the photoluminescent molecules.

In an embodiment, a filter may be arranged in front of the photo-detector to select the range of wavelength that shall be transmitted to the photo-detector. In particular, the filter is used to eliminate the part of the excitation light emitted by the light source that is reflected by the photoluminescent molecules or by the carrier or carrier matrix.

In further embodiments, the carrier can be made as a material comprised in the group of silicone rubber (also known as polydimethylsiloxane), organosubstitute silicones such as poly(R-R'-siloxane) wherein R and R' are one of the following {methyl, ethyl, propyl, butyl, "alkyl", "aryl", phenyl and "vinyl"} and not necessarily equal to each other, polyurethane, poly(1-trimethylsily-1-propyne), polystyrene, poly(methylmethacrylate), poly(vinyl chloride), poly(isobutylmethacrylate), poly(2,2,2-trifluoroethylmethacrylate), ethylcellulose, cellulose acetobutyrate, cellulose acetate, gas-permeable polytetrafluoroethylene and thermoplastic polyurethanes and copolymers, in which material the photoluminescent molecules are embedded. Further, these materials, in particular silicone rubber, thermoplastic polyurethanes and copolymers, gas-permeable polytetrafluoroethylene, are well adapted in the present invention since they are implantable biomaterials. In a particular embodiment, silicone rubber is well adapted if the sensor 1 is used to determine the concentration of oxygen in the region 10 since its permeability to oxygen is about ten times higher than in most polymeric materials, namely 6.95×10¹¹ cm⁻²s⁻¹Pa⁻¹, which corresponds to a diffusion coefficient of about 1.45×10⁻⁵ cm²s⁻¹.

The light source may be a solid state light source, preferentially a light emitting diode, which is advantageous since light emitting diodes are small and can therefore easily be incorporated in the sensor. Light emitting diodes are advantageous since the intensities, wavelengths, and time responses are controllable. Light emitting diodes can emit at various ranges, which generally extend from 390 nm to 1650 nm although not any wavelength of this range may be achieved.

The sensitivity of the photo-detector of the implantable sensor depends on the wavelength range at which the photoluminescent molecules emit. Generally, the range of wavelength at which photoluminescent molecules emit extend from 350 to 1800 nm, in particular in the range of 350-800 nm. Thus, the photo-detector shall be sensitive in this range of wavelength. As the wavelength at which a selected photoluminescent molecules emit is known, the sensitivity of the photo-detector can be selected accordingly. As an example, oxyphors emit light at about 800 nm, which corresponds to near infrared. In this case, the photo-detector may be a commercially available planar InGaAs photodiode, which is sensitive to red and infrared light, i.e. in the spectral range of 800-1800 nm. Such a detector has a response time of up to 120 MHz, which is sufficiently rapid for measuring photoluminescent lifetimes and sufficiently quantitative for steady state measurements of intensities as well. The measurements of lifetimes and intensities will be described in more details later. In particular, the photo-detector may be one of the group comprised of a pn photodiode, a pin photodiode, a Schottky photodiode, an avalanche photodiode, a silicon photodiode, a planar InGaAs photodiode, a SiGe-based optoelectronic circuit and a InGaN/GaN multiple quantum well pn junction.

The photoluminescent molecules may be fluorescent molecules or phosphorescent molecules. Although it would be preferable to use fluorescent molecules as they are generally more stable over time, it is preferable to use phosphorescent molecules as phosphorescence leads to longer timescale, which then facilitates the design of the electronics in the sensor.

Since the sensor is designed to determine the concentration of oxygen in blood, it is preferable that the photoluminescent molecules are not sensitive to other gases than oxygen such as carbon monooxide which can also be found in blood. The photoluminescent molecules shall also be easily bounded to the material of the carrier, i.e. they should be compatible with the characteristics of the carrier.

Photoluminescent molecules that may be used to determined the concentration of oxygen are molecules comprised within the group of pyrene, pyrene derivatives (vinylpirene, methoxypyrene), a polyaromatic carrier, an ionic probe (ruthenium trisbypyridine) and Pd-tetra (4-carboxyphenyl) benzoporphin (Oxyphor). Additional examples are naphthalene derivatives (e.g. 2-dimethylamino-6-lauroylnaphthalene); polypyridyl complexes of transition metals, particularly Ruthenium, Osmium, or Rhenium containing fluorescent molecules {e.g. Ru(bipy)(3)(2+) (tris(2,2'-bipyridyl)ruthenium(II) chloride hexahydrate) and Ru(phen)(3)(2+) (tris(1,10-phenanthroline)ruthenium(II) chloride hydrate)}; fullerenes (C60 and C70); decacyclene; perylene and perylene derivatives (e.g. - perylene dibutylate); and metalloporphines especially Pt(II), Zn(II) and Pd(II) variants.

In yet another embodiment, a sensor for electrochemically measuring the concentration of oxygen in blood is arranged in the device for monitoring progression of heart failure in a human heart according to the present invention. The sensor comprises a working electrode, a reference electrode, and a counter-electrode. The reference and counter electrode can be situated in the pacemaker pocket (subcutaneous). Furthermore, the sensor comprises means for measuring a floating potential at the working electrode, relative to the reference electrode, when the working electrode is in an electrically floating state, and for temporarily retaining the floating potential, means for placing the working electrode at a first potential relative to the reference electrode during a first predetermined measurement period *t*₁ to *t*₂ and thereby causing an electrochemical reaction at the working electrode, means for placing the working electrode at a second potential relative to the reference electrode, equal to the retained floating potential, during a second measurement period *t*₂ to *t*₃ immediately following and equal to the first measurement period, means for identifying a first electrical charge *Q*₁ producing during the first measurement period starting at a time *tᵢ* after *t*₁ with *t*₁ *< tᵢ < t*₂ and for identifying a second electrical charge *Q*₂ of opposite polarity to *Q*₁ durign the second measurement period at the time *tᵢ* after *t*₂ with *t*₂ *< tᵢ < t*₃ ; and means for forming a difference ΔQ by adding *Q*₁ + *Q*₂*,* with ΔQ being proportional to an amount of oxygen in the blood. This sensor is described in more detail in US 6,236,873 which hereby is incorporated by reference in its entirety.

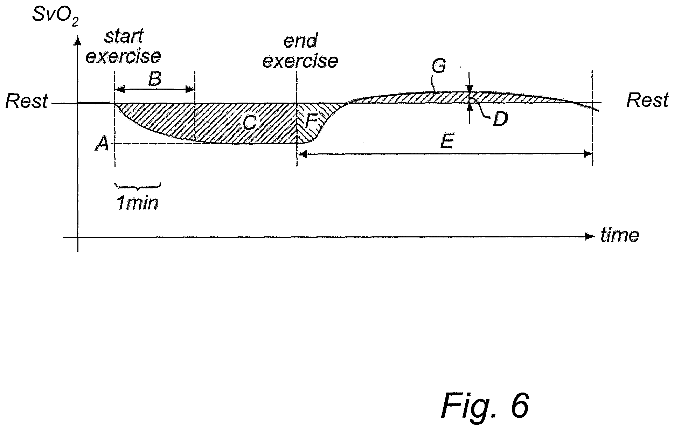

Turning now to Fig. 6, there is schematically illustrated how the SvO₂ level in a patient varies during exercise as compared to the SvO₂ level at rest, denoted in the drawing by the horizontal line marked "Rest". First, the reference characters A through G is explained:
REST Stable SvO₂ value during rest, i.e. no or very low physical activity. Typical value is about 70-75 %. This parameter is decreased with progressing heart failure. However, a detectable change may only be present at more severe degrees of heart failure, such as for groups III and IV according to the NYHA Functional Classification.
A Stable SvO₂ value during exercise. Typical value is about 30-50 %, depending on workload. This parameter is decreased with progressing heart failure.
B The time from onset of activity to new stable SvO₂ level during exercise. Assuming that the workload is constant, the SvO₂ value reaches a new stable level within 1-2 minutes of exercise, which will be maintained until the exercise ends or the level of exercise changes. This time is increased with progressing heart failure.
C The area of the curve during exercise, which depends on length and intensity of exercise. This area is increased at comparable exercise types and lengths with progressing heart failure.
D The maximum overshoot of the SvO₂ level after end of the exercise. Typical value is about 5 %, and is increased with progressing heart failure. Alternatively, the overshoot at a predetermined time after end of the exercise can be measured instead of the maximum overshoot.
E The time from end of the exercise for the SvO₂ value to return to the level at rest, i.e. after a possible overshoot. This time depends on the intensity of exercise and degree of heart failure. An approximate value is in the order of 2-15 minutes. However, this time is increased with progressing heart failure at a specific level of exercise.
F The area between the current SvO₂ value and the resting value from time of end of exercise to return of SvO₂ level to its value at rest, i.e. before a possible overshoot. This area is increased with progressing heart failure at a specific level of exercise.
G The area above the SvO₂ value at rest after the end of exercise, i.e. during the possible overshoot time period. This area is increased with progressing heart failure at a specific level of exercise.

Even though specific SvO₂ parameters has been shown as examples of oxygen signal parameters that could be used for determining degree of heart failure, other parameters are also contemplated. For example, the morphology of the SvO₂ or pO₂ values during and following exercise could be monitored, compared to stored values and evaluated for determining degree of heart failure.

Furthermore, the above parameters should be seen as examples of how SvO₂ data during and after activity can be used to construct clinically useful indices with a better specificity than single SvO₂ values. However, in a device and method of exemplifying embodiments, not all of these parameters are required for determining progression of heart failure in a patient. Thus, any, some or all of the above parameters may be used, including the SvO₂ level at rest. Furthermore, the selection of parameters may be preprogrammed for patients for which embodiments of the invention is implemented, or may be selected by a physician in accordance with the specific heart conditions and needs of a particular patient.

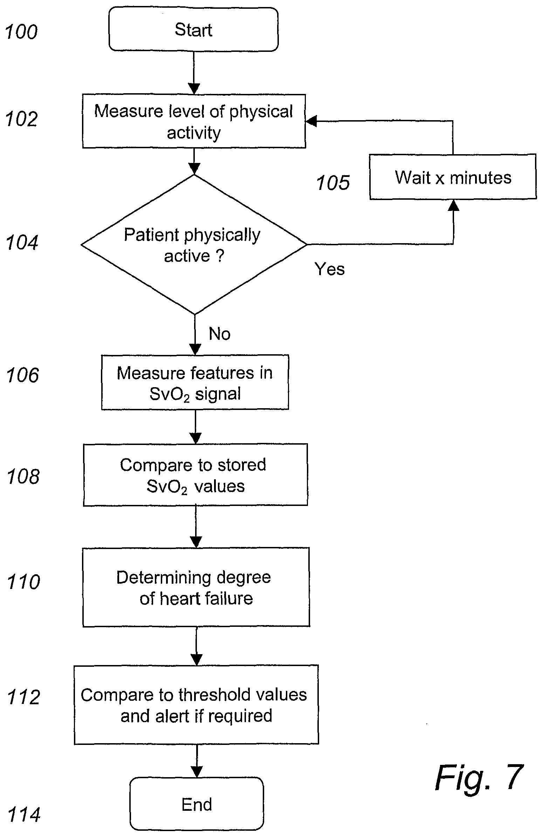

Turning now to the flow diagram shown in Fig. 7, an exemplifying embodiment will be shown. At step 100, a procedure for determining a degree of heart failure is initiated. At 102, a level of physical activity is measured from an activity signal output by an activity sensor. At 104, it is determined whether the patient is physically active or not, i.e. whether the patient is exercising or resting. For the presently described procedure, it is the SvO₂ level at rest that is used for determining the degree of heart failure. Thus, if it is determined at 104 that the patient is exercising or otherwise physically active, then a waiting period is initiated at 105 and completed before a new attempt of determining degree of heart failure is made.

If, on the other hand, the patient is determined to be at rest, the features of the SvO₂ signal is then measured at step 106. In the most general case, a single measurement of the SvO₂ level at rest is performed and used for determining degree of heart failure. Alternatively, the SvO₂ level for a preselected time period is measured for evaluating variations thereof.

Then, the obtained SvO₂ features are at 108 compared to stored values SvO₂ for the measured features. The stored values are preferably historic values obtained at previous measurements for the same patient. The comparison made at 108 is then used at 110 for determining a degree of heart failure. The determined degree of heart failure is also stored, optionally with the measurement results.

Then, at 112, it is also determined whether the determination made at 110 indicates a progressed degree of heart failure by comparing the latest determined degree of heart failure to previous determinations. Thus, if the SvO₂ value at rest has dropped significantly since the subsequent measurement, if the SvO₂ value has fallen below a threshold value, or if latter measurements indicate a negative trend, it can then be determined that the degree of heart failure has deteriorated. If this is the case, an alert signal may be produced, triggering a vibrating alarm to the patient, or an alarm signal to a home monitoring unit or to a clinical site. The alert signal may be an indication that a follow-up is required, or that the patient should seek immediate medical assistance.

In the embodiment of Fig. 7, only SvO₂ values at rest are used for determining the degree of heart failure of a patient. However, as will be apparent from the following description, and is readily understood by the person skilled in the art, the procedure described with reference to Fig. 7 may be used in conjunction with the embodiments to be described below.

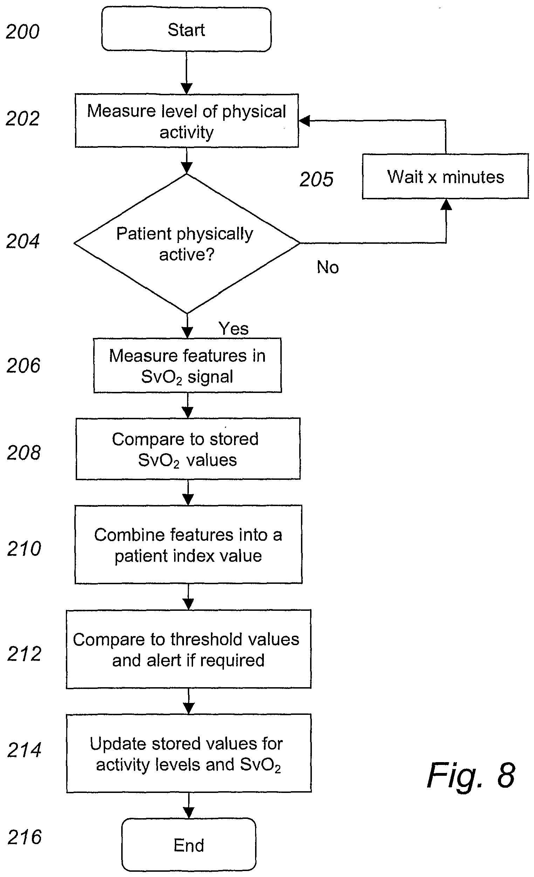

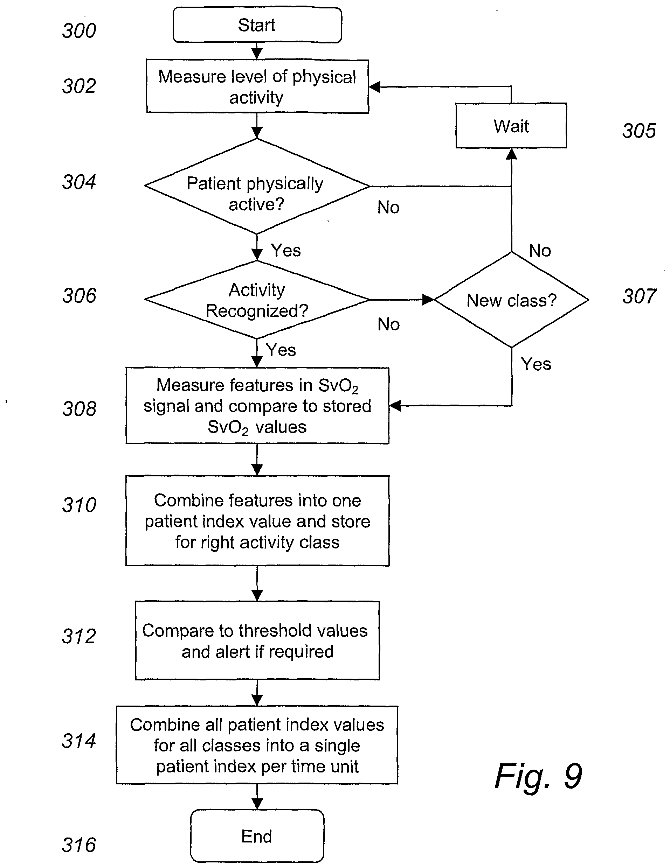

With reference now to Figs. 8 and 9, there will be described exemplifying embodiments using oxygen parameters obtained in mixed venous blood during various levels of exercise for determining a degree of heart failure. First, at 200 the procedure is initiated. Then, at 202, a level of physical activity is measured using the output signal of an activity sensor, such as an accelerometer. At 204, it is determined whether the patient is physically active. In the illustrated embodiment, the procedure is interrupted for a certain time period, at step 205, if it is determined that the physical activity of the patient falls below a given threshold value, i.e. the patient is at rest.

Alternatively, instead of interrupting the procedure at 205, a switch may be performed to the procedure according to Fig. 7 if it is determined that the patient is at rest. In consequence thereof, in the procedure of Fig. 7, a switch to the procedure of Fig. 8 (or of Fig. 9, as will be apparent below) may be actuated once it has been established at 104 that the patient is not at rest.

Then, at step 206, features of the SvO₂ signal is measured and, at 208, compared to stored SvO₂ values. In this embodiment, one or more of the features measured in the SvO₂ signal, for instance in accordance with the listing A through G above, are stored and combined into a single patient index value that correlates to the heart failure status of the patient. As a result of the comparison to threshold values performed at 212, an alert signal may be produced, in the manner described above, indicating that a clinical follow-up is required, or that the patient should seek immediate medical assistance.

The patient index value is then updated at 214, optionally together with both activity levels and SvO₂ levels, and trended over time. Thus, the patient index may be updated each time a measurement is made, which for example can be whenever the activity sensor senses that the patient is physically active. The stored values could then be transmitted to the physician via a home monitoring unit, or simple displayed via a programmer at follow-ups.

Turning to Fig. 9, there will be described a further exemplifying embodiment in which SvO₂ signals obtained during exercise is used for assessing a degree of heart failure. In this example, steps 300-305 are similar to steps 200-205 described above. However, at 306, upon determination that the patient is physically active, characteristics of the activity signal is further evaluated to determine whether the sensed activity is recognized from previous activity measurements. If the answer is no, an interruption period may be initiated and the level of physical activity measured again following the interruption. Alternatively, as illustrated in Fig. 8, a determination may be made at 307 as to whether the sensed activity is to be stored and used for comparison in future activity measurements and ensuing determinations of heart failure degrees. If so, a new class or group of activity is created and stored together with measured SvO₂ values, and the procedure is either returned to measure level of physical activity at 302.

Then, at 308, features of the SvO₂ signal are measured and compared to stored SvO₂ values, which has been obtained through previous measurements on the patient. The features coupled to the recognized group or class of activity are, at 310, combined into a patient index value and stored for the corresponding activity class. By limiting the comparison of SvO₂ parameters to parameters obtained within the same class or group of activity, i.e. at comparable activities, a high sensitivity and specificity is obtained.

A simple classification of activity data could for instance be three classes indicating three different levels of activity, low, moderate and high. A class indicating patient at rest could in further examples be added, as well as more advanced classification procedures involving evaluating morphology of activity signals. Further examples may include adaptive classification of activity levels, as referred to above with reference to step 307, which take into account performed activity measurements and create new activity classes in response to patient behavior.

At 312, a comparison and possible triggering of an alert signal is then performed similar to that described above with reference to Figs. 7 and 8, respectively.

Finally, at 314, all stored patient indices for all patient activity classes may be combined into one single patient index. In this example, one single patient index is stored per time unit, which could for instance be a portion of a day, a full day, a plurality of days, etc., depending on the heart condition and current status of the patient. The combined index, obtained and stored per time unit or otherwise, could then be trended or otherwise stored, processed and transmitted and displayed to a treating physician.

In the above described examples, the measurements have been performed automatically, i.e. without patient interaction. However, it is also within the scope of the present invention to perform measurements on a patient manual basis, or on a semi-automatic basis.

In the example of the patient manually triggering the measurement device to initiate a determination of heart failure degree, the patient could use a home monitoring device or some other extracorporeal means for triggering the device to initiate measurements. Then, the patient could either follow a specific activity scheme, such as walking on a treadmill at a given pace, cycling at a determined pace with a controlled resistance, walking a specified route, doing predefined arm and/or leg movements, etc. The type of activity and length of exercise may be determined by the patient and/or the physician. In one example, the patient chooses a suitable activity, initiates measurements, and tries to perform the same activity for each subsequent measurement, which could for instance be repeated once a day or once a week, depending on the status of the patient. The system could also be taught to recognize and discriminate between different types of activities, while still being triggered to initiate measurements by the patient. The physical activities and measurements could be performed at clinical follow-ups, but is preferably performed at the home of the patient, for instance once a month, week or day. The results of measurements in home environment may then be transmitted to the physician, preferably using a wireless system.

In examples of semi-automatic measurements and degree of heart failure determination, a patient may at a clinical, physician supervised occasion be instructed to perform one or more certain physical activities which are likely to be repeated in every-day life. This would be a calibration process that would teach the implant to recognize certain physical activities. Use can then be made of a suitable activity and/or posture sensor, such as a triaxial accelerometer. In operation, the implant could monitor the physical activities performed by the patient and initiate an SvO₂ measurement and heart failure degree determination whenever the monitored activity concurs with one of the calibrated activities.

While the invention disclosed herein has been described by means of specific embodiments and applications thereof, numerous modifications and variations could be made therein by those skilled in the art without departing from the scope of the invention. Thus, it is to be understood that the above description of the invention and the accompanying drawings is to be regarded as a non-limiting example thereof, and that the scope of protection is defined by the appended claims.

## Claims

1. A medical implant device (20) for monitoring progression of heart failure in a human heart, wherein the device is adapted to be connected to an oxygen sensor (50) and an activity sensor (35), said oxygen sensor (50) being sensitive to a level of venous oxygen content, and arranged to output an oxygen signal, and said activity sensor (35) being sensitive to a level of physical activity of a patient (40) and arranged to output an activity signal, the device comprising processing circuitry arranged for:
receiving an oxygen signal from said oxygen sensor (50),
receiving an activity signal from said activity sensor (35), and
determining the level of physical activity from the received activity signal, **characterized in that** the processing circuitry is further arranged for:
determining whether the level of physical activity is equal to or exceeds a given threshold value;
comparing, if the level of physical activity is equal to or exceeds the given threshold value, a parameter of the received oxygen signal corresponding to a time from onset of the physical activity until a stable oxygen signal during the physical activity or a time from an end of the physical activity during which a stable oxygen level has been reached until the oxygen signal has returned to a normal value to a corresponding stored oxygen signal parameter for the determined level of physical activity, and
determining the degree of heart failure on the basis of said comparison.

2. The device as claimed in claim 1, wherein the processing circuitry is arranged for:
storing compared parameters of the oxygen signal with the corresponding determined level of physical activity, wherein said stored oxygen signal parameter(s) are results of oxygen signal and physical activity levels obtained from previous measurements for the same patient, and
updating said stored oxygen signal parameter(s) in order to monitor the progression of said oxygen signal parameter(s) at corresponding levels of physical activity, thereby monitoring the progression of determined degree of heart failure.

3. The device as claimed in claim 1 or 2, wherein the processing circuitry is arranged for:
determining a present mixed venous oxygen saturation (SvO₂) level by means of the oxygen signal;
determining a time from onset of the physical activity to a new stable SvO₂ level during the physical activity; and
comparing a parameter of the received oxygen signal corresponding to the time from onset of the physical activity to a new stable SvO₂ level during the physical activity to a corresponding stored oxygen signal parameter for the determined level of physical activity.

4. The device as claimed in claim 1 or 2, wherein the processing circuitry is arranged for:
determining a present mixed venous oxygen saturation (SvO₂) level by means of the oxygen signal;
determining a time from the end of the physical activity for the SvO₂ level to return to an SvO₂ level at rest; and
comparing a parameter of the received oxygen signal corresponding to the time from the end of the physical activity for the SvO₂ level to return to the SvO₂ level at rest to a corresponding stored oxygen signal parameter for the determined level of physical activity.

5. The device as claimed in any one of the preceding claims, wherein said processing circuitry is arranged for:
triggering a communication with the patient (40) to instruct the patient (40) to perform a physical activity,
determining that the patient is performing said physical activity, and
performing said determination of physical activity level, said comparison and said determination of degree of heart failure.

6. The device as claimed in claim 5, wherein said processing circuitry is connectable to a telemetry device (37) and arranged for:
triggering a communication via the telemetry device (37) with an extracorporeal communication device (41), such as a home monitoring unit (41).

7. The device as claimed in any one of the preceding claims, wherein said processing circuitry is arranged for:
registering, during a calibration process at the time of implantation or clinical follow-up, a certain activity level of a patient (40) for obtaining a calibrated activity level,
continuously monitoring the level of physical activity for a patient (40) after implantation, and
initiating said comparison when the monitored level of physical activity corresponds to the said calibrated activity level.

8. The device as claimed in any one of the preceding claims, wherein the processing circuitry is arranged for:
monitoring whether said determined degree of heart failure is within one or more predetermined limits, and
triggering a notification action if said determined degree of heart failure falls outside said predetermined limits.

9. The device as claimed in claim 8, wherein the processing circuitry is arranged for:
triggering a transfer of, as an alert action, an alarm signal to an extracorporeal device (41), or to a vibration unit of said medical implant (20) causing it to vibrate.

10. The device as claimed in claim 9, wherein the processing circuitry is arranged for:
triggering a transfer of said alarm signal and/or information related to said determined degree of heart failure and progression thereof to a monitoring device (44) via a network (43) connected to said extracorporeal unit (41), wherein said alarm signal and/or said information can be transferred via said network (43) to the monitoring device (44) located at a care institution connected to said network (43) such that a physician can be informed of a patient status.

11. The device as claimed in any one of the preceding claims, wherein the processing circuitry is arranged for calculating an SvO₂ signal out of said oxygen signal.

12. The device as claimed in any one of the preceding claims, wherein the processing circuitry is arranged for calculating an pO₂ signal.

13. The device as claimed in any one of preceding claims, wherein said oxygen sensor (50) is a pO₂ sensor, or wherein said oxygen sensor (50) is an SvO₂ sensor.

14. The device as claimed in any one of the claims 1-12, wherein said oxygen sensor comprises (50) at least one light source (51a, 51b, 51c) adapted to, during measurement sessions, emit light at least at a first wavelength, at least one photo-detector (54) and at least one type of photoluminescent molecules embedded in a carrier selectively permeable to oxygen, wherein a part of said carrier is in contact with a venous blood of said patient (40), and wherein said at least one light source (51a, 51b, 51c) and said at least one photo-detector (54) are optically connected to said carrier, wherein said photoluminescent molecules emit light in response to excitation by said light emitted from said at least one light source (51a, 51b, 51c) and wherein oxygen reacts with said photoluminescent molecules to alter characteristics of said light emitted from said photoluminescent molecules, wherein said at least one photo-detector (54)is adapted to detect said light emitted from said photoluminescent molecules and to provide output signals representative of said characteristics to determine said concentration of oxygen.

15. The device as claimed in any one of the claims 1-12, wherein said oxygen sensor (50) comprises:
a working electrode;
a reference electrode;
a counter-electrode;
means for measuring a floating potential at said working electrode, relative to said reference electrode, when said working electrode is in an electrically floating state, and for temporarily retaining said floating potential;
means for placing said working electrode at a first potential relative to said reference electrode during a first predetermined measurement period *t₁* to *t₂* and thereby causing an electrochemical reaction at said working electrode;
means for placing said working electrode at a second potential relative to said reference electrode, equal to said retained floating potential, during a second measurement period *t₂* to *t₃* immediately following and equal to said first measurement period;
means for identifying a first electrical charge *Q₁* producing during said first measurement period starting at a time *tᵢ,* after *t₁* with *t₁ < tᵢ < t₂* and for identifying a second electrical charge *Q₂* of opposite polarity to *Q₁* during said second measurement period at said time *tᵢ* after *t₂* with *t₂* < *tᵢ* < *t₃*; and
means for forming a difference ΔQ by adding *Q₁* + *Q₂,* with ΔQ being proportional to an amount of oxygen in said blood.

16. An implantable cardiac stimulator (20) for delivering stimulation pulses to a human heart, comprising:
a pulse generator (25) for generating said stimulation pulses;
control circuitry (27) for controlling the delivery of said stimulation pulses to the heart;
said stimulator (20) being connectable to a cardiac lead arrangement (26a, 26b) for conduction of stimulation pulses to the heart and sensed cardiac electrical activity from the heart, said lead arrangement (26a, 26b) further being arranged for sensing atrial electrical activity and atrial mechanical activity; and
a device as claimed in any one of claims 1-15.

17. The cardiac stimulator as claimed in claim 16, further comprising a telemetry device (37) for communication with an extracorporeal unit (41), such as a programmer or a home monitoring unit (41).

18. The cardiac stimulator as claimed in claim 16 or 17, further comprising a vibration unit for causing said cardiac stimulator to vibrate.

## Patentansprüche

1. Medizinische Implantatvorrichtung (20) zum Überwachen des Fortschreitens von Herzinsuffizienz in einem menschlichen Herzen, wobei die Vorrichtung angepasst ist, um mit einem Sauerstoffsensor (50) und einem Aktivitätssensor (35) verbunden zu sein, wobei der Sauerstoffsensor (50) gegenüber einem Niveau eines venösen Sauerstoffgehalts empfindlich und angeordnet ist, um ein Sauerstoffsignal auszugeben, und wobei der Aktivitätssensor (35) gegenüber einem Niveau von körperlicher Aktivität eines Patienten (40) empfindlich und angeordnet ist, um ein Aktivitätssignal auszugeben, wobei die Vorrichtung eine Verarbeitungsschaltung umfasst, die angeordnet ist zum:
Empfangen eines Sauerstoffsignals von dem Sauerstoffsensor (50), Empfangen eines Aktivitätssignals von dem Aktivitätssensor (35), und
Bestimmen des Niveaus von körperlicher Aktivität aus dem empfangenen Aktivitätssignal,
**dadurch gekennzeichnet, dass** die Verarbeitungsschaltung ferner angeordnet ist zum:
Bestimmen, ob das Niveau von körperlicher Aktivität gleich einem gegebenen Schwellenwert ist, oder diesen überschreitet;
Vergleichen, falls das Niveau von körperlicher Aktivität gleich dem gegebenen Schwellenwert ist, oder diesen überschreitet, eines Parameters des empfangenen Sauerstoffsignals, der einer Zeitspanne von einem Beginn der körperlichen Aktivität bis zu einem stabilen Sauerstoffsignal während der körperlichen Aktivität oder einer Zeitspanne von einem Ende der körperlichen Aktivität, während der ein stabiles Sauerstoffniveau erreicht worden ist, bis das Signal zu einem normalen Wert zurückgekehrt ist, entspricht, mit einem entsprechenden gespeicherten Sauerstoffsignalparameter für das bestimmte Niveau von körperlicher Aktivität, und
Bestimmen eines Grads von Herzinsuffizienz auf der Grundlage des Vergleichs.

2. Vorrichtung nach Anspruch 1, wobei die Verarbeitungsschaltung angeordnet ist, zum:
Speichern von verglichenen Parameters des Sauerstoffsignals mit dem entsprechenden bestimmten Niveau von körperlicher Aktivität, wobei der/die gespeicherte(n) Sauerstoffparameter Ergebnisse von Sauerstoffsignalniveaus und Niveaus von körperlicher Aktivität sind, die aus vorhergehenden Messungen für denselben Patienten erhalten wurden, und
Aktualisieren des/der gespeicherten Sauerstoffsignalparameter(s), um das Fortschreiten des/der Sauerstoffsignalparameter(s) bei entsprechenden Niveaus von körperlicher Aktivität zu überwachen, und damit das Fortschreiten des bestimmten Grads von Herzinsuffizienz zu überwachen.

3. Vorrichtung nach Anspruch 1 oder 2, wobei die Verarbeitungsschaltung angeordnet ist, zum:
Bestimmen eines gegenwärtigen gemischten venösen Sauerstoffsättigungs(SvO₂)-Niveaus mittels des Sauerstoffsignals;
Bestimmen einer Zeit von einem Beginn der körperlichen Aktivität bis zu einem neuen stabilen SvO₂-Niveau während der körperlichen Aktivität; und
Vergleichen eines Parameters des empfangenen Sauerstoffsignals, der einer Zeitspanne von dem Beginn der körperlichen Aktivität bis zu einem neuen stabilen SvO₂-Niveau während der körperlichen Aktivität entspricht, mit einem entsprechenden gespeicherten Sauerstoffsignalparameter für das bestimmte Niveau von körperlicher Aktivität.

4. Vorrichtung nach Anspruch 1 oder 2, wobei die Verarbeitungsschaltung angeordnet ist zum:
Bestimmen eines gegenwärtigen gemischten venösen Sauerstoffsättigungs(SvO₂)-Niveaus mittels des Sauerstoffsignals;
Bestimmen einer Zeit für das SvO₂-Niveau, um von dem Ende der körperlichen Aktivität zu einem SvO₂-Niveau im Ruhezustand zurückzukehren; und
Vergleichen eines Parameters des empfangenen Sauerstoffsignals, der der Zeit entspricht, die das SVO₂-Niveau benötigt, um ab dem Ende der körperlichen Aktivität zu dem SvO₂-Niveau im Ruhezustand zurückzukehren, mit einem entsprechenden gespeicherten Sauerstoffsignalparameter für das bestimmte Niveau von körperlicher Aktivität.

5. Vorrichtung nach einem der vorhergehenden Ansprüche, wobei die Verarbeitungsschaltung angeordnet ist zum:
Auslösen einer Kommunikation mit dem Patienten (40), um den Patienten (40) anzuweisen, eine körperliche Aktivität durchzuführen;
Bestimmen, dass der Patient die körperliche Aktivität durchführt, und
Durchführen des Bestimmens des Niveaus von körperlicher Aktivität, des Vergleichs und des Bestimmens eines Grades von Herzinsuffizienz.

6. Vorrichtung nach Anspruch 5, wobei die Verarbeitungsschaltung mit einer Telemetrievorrichtung (37) verbindbar und angeordnet ist zum:
Auslösen einer Kommunikation über die Telemetrievorrichtung (37) mit einer extrakorporalen Kommunikationsvorrichtung (41), wie einer Heimüberwachungseinheit (41).

7. Vorrichtung nach einem der vorhergehenden Ansprüche, wobei die Verarbeitungsschaltung angeordnet ist zum:
Registrieren, während eines Kalibrierungsverfahrens zur Zeit der Implantation oder einer klinischen Folgemaßnahme, eines gewissen Aktivitätsniveaus eines Patienten (40), um ein kalibriertes Aktivitätsniveau zu erhalten,
kontinuierliches Überwachen des Niveaus von körperlicher Aktivität eines Patienten (40) nach der Implantation, und
Initiieren des Vergleichs, wenn das überwachte Niveau von körperlicher Aktivität dem kalibrierten Aktivitätsniveau entspricht.

8. Vorrichtung nach einem der vorhergehenden Ansprüche, wobei die Verarbeitungsschaltung angeordnet ist zum:
Überwachen, ob der bestimmte Grad an Herzinsuffizienz sich innerhalb von einer oder mehreren vorbestimmten Grenzen befindet, und
Auslösen einer Benachrichtigungsaktion, falls der bestimmte Grad an Herzinsuffizienz außerhalb der vorbestimmten Grenzen liegt.

9. Vorrichtung nach Anspruch 8, wobei die Verarbeitungsschaltung angeordnet ist zum:
Auslösen einer Übertragung, als eine Alarmierungsaktion, eines Alarmsignals an die extrakorporale Vorrichtung (41) oder an eine Vibrationseinheit des medizinischen Implantats (20), das es zum Vibrieren veranlasst.

10. Vorrichtung nach Anspruch 9, wobei die Verarbeitungsschaltung angeordnet ist zum:
Auslösen einer Übertragung des Alarmsignals und/oder von Informationen, die den bestimmten Grad an Herzinsuffizienz und deren Fortschreiten betreffen, an eine Überwachungsvorrichtung (44) über ein Netzwerk (43), das mit der extrakorporalen Einheit (41) verbunden ist, wobei das Alarmsignal und/oder die Informationen über das Netzwerk (43) an die Überwachungsvorrichtung (44) übertragen werden können, die sich in einer Versorgungseinrichtung befindet, die mit dem Netzwerk (43) verbunden ist, sodass ein Arzt über einen Patientenstatus informiert werden kann.

11. Vorrichtung nach einem der vorhergehenden Ansprüche, wobei die Verarbeitungsschaltung zum Berechnen eines SvO₂-Signals aus dem Sauerstoffsignal angeordnet ist.

12. Vorrichtung nach einem der vorhergehenden Ansprüche, wobei die Verarbeitungsschaltung zum Berechnen eines pO₂-Signals angeordnet ist.

13. Vorrichtung nach einem der vorhergehenden Ansprüche, wobei der Sauerstoffsensor (50) ein pO₂-Sensor ist, oder wobei der Sauerstoffsensor (50) ein SvO₂-Sensor ist.

14. Vorrichtung nach einem der Ansprüche 1-12, wobei der Sauerstoffsensor (50) wenigstens eine Lichtquelle (51a, 51b, 51c), die angepasst ist, um, während einer Messsitzung Licht von wenigstens einer ersten Wellenlänge auszustrahlen, wenigstens einen Photodetektor (54) und wenigstens eine Art von photolumineszierenden Molekülen umfasst, die in ein Trägermaterial eingebettet sind, das selektiv permeabel für Sauerstoff ist, wobei ein Teil des Trägermaterials in Kontakt mit venösem Blut des Patienten (40) ist, und wobei die wenigstens eine Lichtquelle (51a, 51b 51c) und der wenigstens eine Photodetektor (54) optisch mit dem Trägermaterial verbunden sind, wobei die photolumineszierenden Moleküle als Reaktion auf eine Erregung durch das Licht, das von der wenigstens einen Lichtquelle (51a, 51b, 51c) ausgestrahlt wird, Licht ausstrahlen, und wobei Sauerstoff mit den photolumineszierenden Molekülen reagiert, um Merkmale des Lichts zu ändern, das von den photolumineszierenden Molekülen ausgestrahlt wird, wobei der wenigstens eine Photodetektor (54) angepasst ist, um das Licht zu erfassen, das von den photolumineszierenden Molekülen ausgestrahlt wird, und Ausgabesignale bereitzustellen, die die Merkmale darstellen, um die Sauerstoffkonzentration zu bestimmen.

15. Vorrichtung nach einem der Ansprüche 1-12, wobei der Sauerstoffsensor (50) Folgendes umfasst:
eine Arbeitselektrode;
eine Referenzelektrode;
eine Zählerelektrode;
Mittel zum Messen eines Floatingpotenzials an der Arbeitselektrode relativ zu der Referenzelektrode, wenn die Arbeitselektrode sich in einem elektrischen Floatingzustand befindet, und zum zeitweisen Zurückhalten des Floatingpotenzials;
Mittel zum Platzieren der Arbeitselektrode an einem ersten Potenzial relativ zu der Referenzelektrode während einer ersten vorbestimmten Messperiode *t₁* bis *t₂* und dadurch Veranlassen einer elektrochemischen Reaktion an der Arbeitselektrode;
Mittel zum Platzieren der Arbeitselektrode an einem zweiten Potenzial relativ zu der Referenzelektrode, das dem zurückgehaltenen Floatingpotenzial gleich ist, während einer zweiten Messperiode t₂ bis t₃, die unmittelbar auf die erste Messperiode folgt und dieser gleich ist;
Mittel zum Identifizieren einer ersten elektrischen Ladung *Q₁* während der ersten Messperiode, die bei einer Zeit *tᵢ* nach *t₁* beginnt, wobei *t₁*<*tᵢ*<*t₂*, und zum Identifizieren einer zweiten elektrischen Ladung *Q₂* mit umgekehrter Polarität gegenüber *Q₁* während der zweiten Messperiode bei dem Zeitpunkt *tᵢ* nach *t₂*,
wobei *t₂*<*tᵢ*<*t₃*; und
Mittel zum Bilden einer Differenz ΔQ durch ein Addieren von *Q₁*+*Q₂,* wobei ΔQ proportional zu einer Sauerstoffmenge in dem Blut ist.

16. Implantierbarer Herzschrittmacher (20) zum Liefern von Stimulationsimpulsen an ein menschliches Herz, der Folgendes umfasst:
einen Impulsgenerator (25) zum Erzeugen der Stimulationsimpulse;
Steuerschaltung (27) zum Steuern der Lieferung der Stimulationsimpulse an das Herz;
wobei der Schrittmacher (20) mit einer Herzleitungsanordnung (26a, 26b) für eine Leitung von Stimulationsimpulsen an das Herz und erfasster elektrischer Herzaktivität von dem Herzen weg verbindbar ist, wobei die Leitungsanordnung (26a, 26b) ferner zum Erfassen einer arteriellen elektrischen Aktivität und einer arteriellen mechanischen Aktivität angeordnet ist; und
eine Vorrichtung nach einem der Ansprüche 1-15.

17. Herzschrittmacher nach Anspruch 16, ferner umfassend eine Telemetrievorrichtung (37) zur Kommunikation mit einer extrakorporalen Einheit (41), wie einer Programmiereinrichtung oder einer Heimüberwachungseinheit (41).

18. Herzschrittmacher nach Anspruch 16 oder 17, ferner umfassend eine Vibrationseinheit, um den Herzschrittmacher zu veranlassen zu vibrieren.

## Revendications

1. Dispositif d'implant médical (20) pour surveiller la progression d'une insuffisance cardiaque dans un coeur humain, dans lequel le dispositif est adapté à être relié à un capteur d'oxygène (50) et un capteur d'activité (35), ledit capteur d'oxygène (50) étant sensible à un niveau de contenu d'oxygène veineux, et agencé pour produire un signal d'oxygène, et ledit capteur d'activité (35) étant sensible à un niveau d'activité physique d'un patient (40) et agencé pour produire un signal d'activité, le dispositif comprenant un circuit de traitement agencé pour :
recevoir un signal d'oxygène dudit capteur d'oxygène (50),
recevoir un signal d'activité dudit capteur d'activité (35), et
déterminer le niveau d'activité physique à partir du signal d'activité reçu,
**caractérisé en ce que** le circuit de traitement est en outre agencé pour :
déterminer si le niveau d'une activité physique est égal à une valeur seuil donnée ou l'excède ;
comparer, si le niveau d'activité physique est égal à la valeur seuil donnée ou l'excède, un paramètre du signal d'oxygène reçu correspondant à une durée du début de l'activité physique jusqu'à un signal d'oxygène stable durant l'activité physique ou une durée d'une fin de l'activité physique durant laquelle un niveau d'oxygène stable a été atteint jusqu'à ce que le signal d'oxygène retourne à une valeur normale, à un paramètre de signal d'oxygène stocké correspondant pour le niveau déterminé d'activité physique, et
déterminer le degré d'insuffisance cardiaque sur la base de ladite comparaison.

2. Dispositif selon la revendication 1, dans lequel le circuit de traitement est agencé pour :
stocker des paramètres comparés du signal d'oxygène avec le niveau déterminé correspondant d'activité physique, dans lequel ledit (lesdits) paramètre(s) de signal d'oxygène stocké(s) sont les résultats du signal d'oxygène et des niveaux d'activité physique obtenus de mesures préalables pour le même patient, et mettre à jour ledit (lesdits) paramètre(s) de signal d'oxygène stocké(s) de manière à surveiller la progression dudit (desdits) paramètre(s) de signal d'oxygène à des niveaux correspondants d'activité physique, permettant ainsi de surveiller la progression d'un degré déterminé d'insuffisance cardiaque.

3. Dispositif selon la revendication 1 ou 2, dans lequel le circuit de traitement est agencé pour :
déterminer un niveau présent de saturation en oxygène veineux mixte (SvO₂) au moyen du signal d'oxygène ;
déterminer une durée du début de l'activité physique à un nouveau niveau stable de SvO2 pendant l'activité physique ; et
comparer un paramètre du signal d'oxygène reçu correspondant à la durée du début de l'activité physique à un nouveau niveau stable de SvO₂ durant l'activité physique à un paramètre de signal d'oxygène stocké correspondant pour le niveau déterminé d'activité physique.

4. Dispositif selon la revendication 1 ou 2, dans lequel le circuit de traitement est agencé pour :
déterminer un niveau présent de saturation en oxygène veineux mixte (SvO₂) au moyen du signal d'oxygène ;
déterminer une durée de la fin de l'activité physique au retour du niveau de SvO₂ à un niveau de SvO₂ au repos ; et
comparer un paramètre du signal d'oxygène reçu correspondant à la durée de la fin de l'activité physique au retour du niveau de SvO₂ à un niveau de SvO₂ au repos à un paramètre de signal d'oxygène stocké correspondant pour le niveau déterminé d'activité physique.

5. Dispositif selon l'une quelconque des revendications précédentes, dans lequel ledit circuit de traitement est agencé pour :
déclencher une communication avec le patient (40) pour donner au patient (40) l'instruction de réaliser une activité physique,
déterminer que le patient réalise ladite activité physique, et réaliser ladite détermination du niveau d'activité physique, ladite comparaison et ladite détermination du degré d'insuffisance cardiaque.

6. Dispositif selon la revendication 5, dans lequel ledit circuit de traitement peut être relié à un dispositif de télémétrie (37) et agencé pour :
déclencher une communication via le dispositif de télémétrie (37) avec un dispositif de communication extracorporel (41), tel qu'une unité de surveillance à domicile (41).

7. Dispositif selon l'une quelconque des revendications précédentes, dans lequel ledit circuit de traitement est agencé pour :
enregistrer, durant un processus d'étalonnage au moment de l'implantation ou du suivi clinique, un certain niveau d'activité d'un patient (40) pour obtenir un niveau d'activité étalonné,
surveiller en continu le niveau d'activité physique d'un patient (40) après l'implantation, et
initier ladite comparaison lorsque le niveau surveillé d'activité physique correspond au dit niveau d'activité étalonné.

8. Dispositif selon l'une quelconque des revendications précédentes, dans lequel le circuit de traitement est agencé pour :
surveiller si ledit degré déterminé d'insuffisance cardiaque se trouve dans une ou plusieurs limites prédéterminées, et
déclencher une action de notification si ledit degré déterminé d'insuffisance cardiaque tombe hors desdites limites prédéterminées.

9. Dispositif selon la revendication 8, dans lequel le circuit de traitement est agencé pour :
déclencher un transfert, en tant qu'action d'alerte, d'un signal d'alarme à un dispositif extracorporel (41), ou à une unité de vibration dudit implant médical (20) provoquant sa vibration.

10. Dispositif selon la revendication 9, dans lequel le circuit de traitement est agencé pour :
déclencher un transfert dudit signal d'alarme et/ou desdites informations liées au dit degré déterminé d'insuffisance cardiaque et à sa progression à un dispositif de surveillance (44) via un réseau (43) relié à ladite unité extracorporelle (41), dans lequel ledit signal d'alarme et/ou lesdites informations peuvent être transférés via ledit réseau (43) au dispositif de surveillance (44) situé au niveau d'un établissement de soins relié au dit réseau (43) de telle sorte qu'un médecin peut être informé d'un statut patient.

11. Dispositif selon l'une quelconque des revendications précédentes, dans lequel le circuit de traitement est agencé pour calculer un signal de SvO₂ hors dudit signal d'oxygène.

12. Dispositif selon l'une quelconque des revendications précédentes, dans lequel le circuit de traitement est agencé pour calculer un signal de pO₂.

13. Dispositif selon l'une quelconque des revendications précédentes, dans lequel ledit capteur d'oxygène (50) est un capteur de pO₂, ou dans lequel ledit capteur d'oxygène (50) est un capteur de SvO₂.

14. Dispositif selon l'une quelconque des revendications 1 à 12, dans lequel ledit capteur d'oxygène (50) comprend au moins une source de lumière (51a, 51b, 51c) adaptée à, durant les sessions de mesure, émettre une lumière au moins à une première longueur d'onde, au moins un photo-détecteur (54) et au moins un type de molécules photoluminescentes intégrées dans un vecteur sélectivement perméable à l'oxygène, dans lequel une partie dudit vecteur est en contact avec un sang veineux dudit patient (40), et dans lequel ladite au moins une source de lumière (51a, 51b, 51c) et ledit au moins un photo-détecteur (54) sont optiquement reliés au dit vecteur, dans lequel lesdites molécules photoluminescentes émettent de la lumière en réponse à l'excitation par ladite lumière émise par ladite au moins une source de lumière (51a, 51b, 51c) et dans lequel l'oxygène réagit avec lesdites molécules photoluminescentes pour modifier les caractéristiques de ladite lumière émise par lesdites molécules photoluminescentes, dans lequel ledit au moins un photo-détecteur (54) est adapté à détecter ladite lumière émise par lesdites molécules photoluminescentes et à fournir des signaux de sortie représentatifs desdites caractéristiques pour déterminer ladite concentration en oxygène.

15. Dispositif selon l'une quelconque des revendications 1 à 12, dans lequel ledit capteur d'oxygène (50) comprend :
une électrode de travail ;
une électrode de référence ;
une contre-électrode ;
un moyen pour mesurer un potentiel flottant au niveau de ladite électrode de travail, relativement à ladite électrode de référence, lorsque ladite électrode de travail est dans un état électriquement flottant, et pour temporairement retenir ledit potentiel flottant ;
un moyen pour placer ladite électrode de travail à un premier potentiel relativement à ladite électrode de référence durant une première période de mesure prédéterminée t₁ à t₂ et ainsi provoquer une réaction électrochimique au niveau de ladite électrode de travail ;
un moyen pour placer ladite électrode de travail à un second potentiel relativement à ladite électrode de référence, égal au dit potentiel flottant conservé, durant une seconde période de mesure t₂ à t₃ suivant immédiatement et égale à ladite première période de mesure,
un moyen pour identifier une première charge électrique Q₁ produisant durant ladite première période de mesure commençant à un moment tᵢ, après t₁ avec t₁ < tᵢ < t₂ et pour identifier une seconde charge électrique Q₂ de polarité opposée à Q₁ durant la seconde période de mesure à un moment tᵢ après t₂ avec t₂ < tᵢ < t₃ ; et
un moyen de formation d'une différence ΔQ par ajout de Q₁ + Q₂, ΔQ étant proportionnelle à une quantité d'oxygène dans ledit sang.

16. Stimulateur cardiaque implantable (20) pour délivrer des impulsions de stimulation à un coeur humain, comprenant :
un générateur d'impulsions (25) pour générer lesdites impulsions de stimulation ;
un circuit de commande (27) pour commander la délivrance desdites impulsions de stimulation au dit coeur ;
ledit stimulateur (20) pouvant être relié à un agencement de dérivation cardiaque (26a, 26b) pour la conduction d'impulsions de stimulation au coeur et l'activité électrique cardiaque détectée du coeur, ledit agencement de dérivation (26a, 26b) étant en outre agencé pour détecter l'activité électrique auriculaire et l'activité mécanique auriculaire ; et
un dispositif selon l'une quelconque des revendications 1 à 15.

17. Stimulateur cardiaque selon la revendication 16, comprenant en outre un dispositif de télémétrie (37) pour la communication avec une unité extracorporelle (41), tel qu'un programmateur ou une unité de surveillance à domicile (41).

18. Stimulateur cardiaque selon la revendication 16 ou 17, comprenant en outre une unité de vibration pour entraîner la vibration dudit stimulateur cardiaque.
